(19) Europäisches Patentamt
European Patent Office
Office européen des brevets

(11) EP 1 350 517 B1

(12) # FASCICULE DE BREVET EUROPEEN

(45) Date de publication et mention de la délivrance du brevet:
**21.09.2011 Bulletin 2011/38**

(21) Numéro de dépôt: **03368024.0**

(22) Date de dépôt: **28.03.2003**

(51) Int Cl.:
***A61K 36/02*** *(2006.01)* ***A61K 8/44*** *(2006.01)*
***A61K 8/64*** *(2006.01)* ***A61Q 19/08*** *(2006.01)*
***A61P 17/00*** *(2006.01)*

(54) **Adduit algal extrait de macroalgues rouges comprenant le dipeptide citrullinylarginine, son procédé d'obtention et son utilisation dermatologique comme agent cosmétique**

Algenaddukt, extrahiert von roten Makroalgen beinhaltend das Dipeptid Citrullinyl-Arginin, sein Herstellungsverfahren sowie seine dermale Verwendung als kosmetische Substanz

Algal adduct extracted from red macro algae containing the dipeptide citrullinyl-arginine, its process of obtaining and its dermatological use as a cosmetic agent

(84) Etats contractants désignés:
**AT BE BG CH CY CZ DE DK EE ES FI FR GB GR HU IE IT LI LU MC NL PT RO SE SI SK TR**

(30) Priorité: **05.04.2002 MC 2484**

(43) Date de publication de la demande:
**08.10.2003 Bulletin 2003/41**

(73) Titulaire: **EXSYMOL S.A.M.**
**MC-98000 Monte Carlo (MC)**

(72) Inventeur: **Seguin, Marie-Christine**
**98000 Monaco (MC)**

(74) Mandataire: **de Zeeuw, Johan Diederick**
**Murgitroyd & Company**
**Immeuble Atlantis**
**55, Allée Pierre Ziller**
**Sophia Antipolis**
**06560 Valbonne (FR)**

(56) Documents cités:
**EP-A- 1 060 739 FR-A- 2 655 268**

- **DATABASE BIOSIS [en ligne] BIOSCIENCES INFORMATION SERVICE, PHILADELPHIA, PA, US; 1977 ITO K ET AL: "AMINO-ACID COMPOSITION OF THE ETHANOLIC EXTRACTIVES FROM 31 SPECIES OF MARINE RED ALGAE" Database accession no. PREV197866036288 XP002247061 & JOURNAL OF THE FACULTY OF FISHERIES AND ANIMAL HUSBANDRY HIROSHIMA, vol. 16, no. 1, 1977, pages 77-90, 1977 JA ISSN: 0440-8756**
- **DATABASE BIOSIS [en ligne] BIOSCIENCES INFORMATION SERVICE, PHILADELPHIA, PA, US; 1977 LAYCOCK M V ET AL: "THE OCCURRENCE AND SEASONAL VARIATION OF GIGARTININE AND L CITRULLINYL-L ARGININE IN CHONDRUS-CRISPUS" Database accession no. PREV197763064793 XP002247062 & CANADIAN JOURNAL OF BIOCHEMISTRY, vol. 55, no. 1, 1977, pages 27-30, ISSN: 0008-4018**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 350 517 B1

## Description

La présente invention a pour objet un adduit algal du dipeptide naturel citrullinylarginine, son utilisation non-thérapeutique dermatologique comme agent de soin et de traitement pour la peau et les phanères.

La littérature rapporte la propriété commune aux macroalgues de consommer l'azote des sources externes présentes dans la mer sous forme d'ions nitrate, nitrite, ammonium, de le réduire et enfin de le stocker sous forme organique, notamment en acides aminés, protéines et pigments (Hanisak M.D.(1983), *In* Carpenter, E.J. and Capone, D.G.(Eds) "Nitrogen in the marine environment". Academic press Inc.).

Ainsi, dans un extrait d'algue rouge *Grateloupia turuturu* (Miyazawa K. et al., Bulletin of the Japonese Society of Scientific Fisheries (1974), vol.40, pp.815-818), il fut isolé et caractérisé structuralement pour la première fois un modèle organique azoté sous la forme du dipeptide citrullinylarginine. D'autres rhodophycées telles *Grateloupia filicina, Polyides rotundus, Polysiphonia lanosa* ont par la suite été identifiées avec la présence du même dipeptide dans la composition de leurs extraits alcooliques, et tout particulièrement l'algue *Chondrus crispus*. Chez cette dernière par exemple, lorsque récoltée au large du Canada et à la fin de la période hivernale, le dipeptide citrullinylarginine constitue le principal modèle organique azoté et représente plus de 50% de l'azote total présent dans la plante (Laycock M.V. et al., Can J. Biochem. (1977), vol.55, pp.27-30).

Par la suite, de nouvelles études menées par le même groupe de chercheurs canadiens sur l'algue *Chondrus crispus* ont toutefois mis en évidence l'influence d'autres facteurs physiologiques que la biodisponibilité en azote des eaux côtières canadiennes dans les conditions de formation naturelle du dipeptide citrullinylarginine dans l'algue (Laycock M.V. et al., Can J. Biochem. (1981), vol.59, pp.522-527). Ils ont ainsi constaté que les teneurs en citrullinylarginine fluctuaient au cours de l'année, avec un processus d'accumulation de la molécule essentiellement pendant l'hiver alors que la température de l'eau est inférieure à 15 DEG C. La concentration maximale en dipeptide est d'ailleurs observée au sortir de l'hiver. Les mêmes auteurs soulignent également, lors de ce processus, le stade végétatif de la plante, sa croissance étant limitée par les eaux froides. Enfin, un minimum d'irradiance ou d'intensité lumineuse s'avère aussi un élément indispensable audit processus.

Les auteurs des travaux cités plus hauts n'avancent toutefois, pour le dipeptide accumulé et stocké, qu'un rôle de réserve potentielle d'azote, énergétique pour la plante. Cette réserve est en effet mise à disposition puis consommée rapidement par celle-ci dès que les concentrations en sources azotées externes dans la mer s'appauvrissent, notamment avec le retour du printemps. Le dipeptide soutient alors la plante et lui permet de profiter pleinement des conditions favorables à sa croissance.

La demande de brevet français N° 2 655 268 décrit les propriétés anti-radicalaires d'extraits d'algues vertes, brunes ou rouge et leurs utilisations dans des domaines variées dont pharmaceutique et cosmétiques.

L'utilisation dermatologique par la demanderesse du dipeptide citrullinylarginine comme agent de soin et de traitement pour la peau et les phanères a été envisagée pour plusieurs raisons.

Tout d'abord, la littérature ne rapporte pas, au sein des tissus cutanés et des phanères des mammifères supérieurs, la biosynthèse d'un tel apport favorable sous cette forme. Ensuite, de par la structure et le nombre d'atomes d'azote du dipeptide citrullinylarginine, un tel apport est susceptible de constituer, à l'instar de son comportement observé chez l'algue, une source d'azote importante et intéressante par exemple à la peau dans le processus de cicatrisation et de réparation des tissus cutanés puisqu'une perte d'azote est classiquement observée au cours de la période post-traumatique (Chyun J.H. et al., J. Nutr. (1984), vol.114, pp.1697-1704). Il est également visé les problèmes d'acidose d'une peau perturbée et le maintien d'un pH neutre intracellulaire avec une telle source d'azote.

L'intérêt du dipeptide comme agent de soin et de traitement résulte également de ses propriétés biologiques, découvertes par la demanderesse et exposées dans la description détaillée de l'invention ci-après. L'action favorable du dipeptide citrullinylarginine sur le métabolisme énergétique de fibroblastes en culture et un comportement cytostimulant ont par exemple été mis en évidence, respectivement par un dosage de l'adénosine triphosphate et un test d'évaluation de la prolifération cellulaire.

Corrélativement au rôle physiologique tenu par le dipeptide dans l'algue et sur la base d'une surexpression connue des protéines chaperonnes (*Heat Shock Proteins* ou HSP) en réponse à un choc thermique négatif (Holland D.B. et al., J. Invest. Dermatology (1993), vol.101, pp.196-199), la demanderesse a démontré l'intérêt d'utiliser le dipeptide au niveau cutané pour répondre au besoin de traitement d'une peau soumise au froid. Dans ces conditions défavorables, la réserve énergétique constituée par la molécule citrullinylarginine améliore la synthèse des protéines au niveau des cellules épidermiques et permet de favoriser l'expression de ces protéines chaperonnes.

La demanderesse a également mis en évidence l'intérêt du dipeptide pour répondre au besoin de traitement d'une peau soumise à une luminosité réduite sans accentuation de l'atonicité ni de la perte d'éclat cutané.

Par ailleurs, de façon plus secondaire, il est par exemple également envisageable d'utiliser le dipeptide citrullinylarginine comme source d'arginine et de citrulline, sous l'action des protéases de la peau au fur et à mesure de sa pénétration cutanée. Un apport exogène de ces deux aminoacides et notamment d'arginine est aussi aujourd'hui d'un intérêt recherché en dermatologie. L'arginine est ainsi fortement impliqué dans le processus de cicatrisation en raison

de son caractère antioxydant. Il est aussi décrit comme substrat pour la synthèse de collagène (Chithra P. et al., J. Clin. Biochem. Nutr. (1995), vol.18, pp.111-117 et références citées).
La présence de la citrulline est retrouvée dans des protéines épidermiques (Kubilus J. et al., Biochim. Biophys. Acta (1979), vol.581, pp.114-121) et dans des protéines capillaires (Rogers G.E. et al., Biochim. Biophys. Acta (1977), vol. 495, pp.159-175), après une conversion enzymatique des résidus arginine latéraux. L'arginine et la citrulline sont connues comme des intermédiaires métaboliques du cycle de l'urée. Une production accrue d'urée endogène est notamment recherchée dans les problèmes de peau sèche (Wohlrab J., Skin Pharmacol. Appl. Skin Physiol. (2002), vol.15, pp.44-54).

Toutefois, de manière inattendue et fort surprenante, la demanderesse a constaté le potentiel génotoxique dudit dipeptide, tant pour la substance purifiée extraite de l'algue *Chondrus crispus* que pour une réplique obtenue par synthèse chimique.
Ces deux résultats sont le fruit d'investigations réalisées par la demanderesse selon les lignes directrices de l'OCDE 471, dans le respect d'une méthode aujourd'hui bien établie dans la recherche du potentiel mutagène pour toute forme administrable chez l'homme : la méthode d'Ames (Kirkland D.J., Mutation Research (1994), vol.312, pp.195-199), (ICH steering committee, 19 Juillet 1995, Guidance on specific aspect of regulatory genotoxicity tests for pharmaceuticals).

Il est bien évident qu'un tel comportement est absolument incompatible avec toute utilisation en dermatologie du dipeptide citrullinylarginine.

Les inventeurs de la présente invention ont alors poursuivi leurs recherches avec des travaux orientés vers l'obtention de formes de citrullinylarginine dépourvues de potentiel génotoxique, tout en conservant l'ensemble des activités originellement démontrées pour le dipeptide.

La demanderesse a ainsi identifié différents dipeptides de synthèse, fonctionnalisés partiellement ou totalement sur trois de ses sites possibles de substitution, doués des mêmes propriétés biologiques que celles de la substance naturelle purifiée. Par contre, les structures identifiées comme telles n'ont pas présenté l'effet secondaire inacceptable susmentionné.

Parallèlement, la demanderesse a observé la suppression du caractère génotoxique pour un extrait algal, enrichi en polypeptides ou protéines, et obtenu selon le protocole ci-après mis au point par les inventeurs de la présente invention.

L'invention a donc pour premier objet l'obtention d'un analogue du dipeptide naturel, analogue lui aussi d'origine algale mais dans lequel il n'est plus retrouvé le potentiel génotoxique affèrent à la molécule naturelle purifiée. Les propriétés biologiques remarquées chez cette dernière sont cependant intégralement conservées.

La première étape du protocole est une extraction d'algues, notamment de macroalgues rouges, préférentiellement de *Chondrus crispus,* à l'aide d'un solvant ou d'un mélange de solvants acceptables en pharmacie.

Ladite algue est préférablement une algue ayant accumulée de façon optimale 10% en poids sec de citrullinylarginine par rapport au poids total d'algue sèche.

Le procédé d'extraction utilisable pour obtenir l'extrait objet de l'invention est avantageusement mis en oeuvre dans les conditions telles que ladite extraction est réalisée au reflux dudit solvant ou mélange de solvants, pendant une durée de 2 à 4 heures.

A titre de solvant préféré pour ladite extraction, on utilisera de préférence un mélange d'eau et d'éthanol ammoniacal.

La seconde étape vise à enrichir la quantité en poids de polypeptides ou protéines de nature intrinsèque jusqu'à une teneur totale au moins égale à 20%.

Suivant un mode avantageux de réalisation de l'invention, l'enrichissement résulte d'une concentration des protéines natives intrinsèques. Alternativement, l'enrichissement peut être extrinsèque et résulte de l'incorporation de protéines standards utilisables en pharmacie.

Préférentiellement pour ces protéines incorporées, on utilisera des protéines végétales ou leurs hydrolysats, tels que notamment les protéines de blé ou de soja.

Préférentiellement pour ces protéines incorporées, tout en tenant compte de la réglementation en vigueur, on utilisera des protéines animales ou leurs hydrolysats, tels que notamment le collagène ou l'élastine.

Préférentiellement pour ces protéines incorporées, on utilisera du collagène marin ou son hydrolysat.

Préférentiellement pour ces protéines incorporées, on utilisera d'autres protéines algales ou leurs hydrolysats, tels que notamment la spiruline et les protéines microalgales.

Eventuellement à l'extrait obtenu peut être incorporé une teneur titrée du dipeptide citrullinylarginine obtenu par voie chimique, de manière à supplémenter ledit extrait jusqu'à 10% en poids.

La troisième étape est une étape de traitement à chaud dudit extrait enrichi en polypeptides ou protéines.

Ledit traitement est avantageusement réalisé durant 3 heures à 40°C.

Indéniablement, au regard de sa réponse négative au test d'Ames, ledit analogue issu du protocole ci-dessus décrit constitue un produit structuralement nouveau, différent de la molécule naturelle purifiée, probablement par son état d'adduit et l'existence de nombreuses intéractions avec lesdits polypeptides ou protéines.

Un deuxième objet de l'invention concerne l'utilisation de cet adduit algal, qualifié plus communemment d'extrait,

comme agent de soin et de traitement au sens de la présente invention des agents qui présentent, de façon générale, une activité réparatrice et revitalisante leur permettant entre autres de mieux réagir aux agressions telles que le froid ou l'obscurité.

La demande internationale de brevet publiée sous le n° WO94/09750 fait état d'une composition cosmétique à base de dérivés peptidiques de citrulline et d'arginine. Des dérivés dipeptidiques y sont même avantageusement divulgués. Les structures exemplifiées sont toutefois différentes de celles objets de l'invention en raison d'une position différente de la liaison peptidique, ainsi que les propriétés recherchées.

L'utilisation cosmétique d'analogues chimiques de citrullinylarginine a certes été déjà décrite dans une demande de brevet déposée par la demanderesse et publiée sous le n° EP 1 060 739 A1. Elle divulgue une composition cosmétique pour l'amincissement à base de L-arginine, d'un analogue de L-arginine ou d'un de leurs dérivés, applicable par voie topique. Mais à nouveau, les propriétés recherchées au niveau cutané et des phanères par les mêmes analogues selon l'invention ne sont pas mentionnées ni même suggérées dans ce document antérieur.

La recherche d'une relation structure/activité, menée par la demanderesse sur lesdits analogues chimiques, a révélé qu'une condition essentielle à la suppression du potentiel génotoxique est la substitution de la fonction α-amino de l'unité citrulline.

La substitution des fonctions α-carboxy et guanido de l'unité arginine a elle aussi été envisagée. Tout en conservant ce caractère de non toxicité, elle apparait toutefois plus accessoire en permettant plutôt une amélioration de l'efficacité desdits analogues aux fins revendiquées.

C'est pourquoi il est également décrit l'utilisation dermatologique d'analogues du dipeptide naturel citrullinylarginine, ou l'un quelconque de leurs sels, comme agents de soin et de traitement pour la peau et les phanères, lesdits analogues étant représentés par la formule générale (I) suivante :

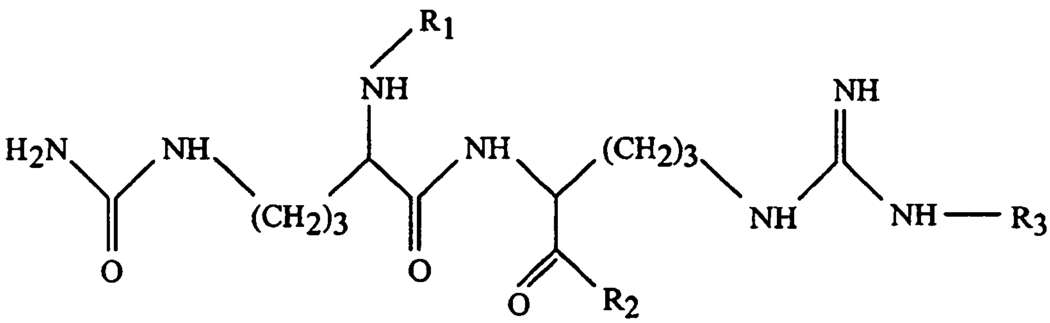

(I)

dans laquelle

$R_1$ représente un radical acyle ou acyloxy,

$R_2$ représente un radical hydroxyle, amine, alkylamine ou alcoxy,

et $R_3$ représente un atome d'hydrogène ou un radical hydroxyle.

A l'instar de l'analogue d'origine algale précédemment décrit, tous ces dérivés ne sont pas potentiellement génotoxiques. Leur usage est, par conséquent, totalement acceptable en dermatologie.

Ces dérivés sont le plus simplement synthétisés suivant des méthodes connues de l'homme du métier, par exemple par acylation de la fonction α-amino et/ou par estérification de la fonction α-carboxylique.

Suivant un mode de réalisation préféré, le composé de formule générale (I) est tel que $R_1$ est un radical acyle ou acyloxy, avantageusement un radical acétyle, R2 est un radical alcoxy, avantageusement un radical éthyloxy, et $R_3$ est un atome d'hydrogène.

Un autre mode de réalisation préféré est d'utiliser un composé de formule générale (I) dans lequel $R_1$ est un radical acyle ou acyloxy, avantageusement un radical acétyle, R2 est un radical hydroxyle et $R_3$ est un atome d'hydrogène.

Les exemples ci-après constituent une liste desdits analogues

N-acetyl-L-citrullinyl-L-arginine ethyl ester (NAc-L-CIT-L-ARG-OEt)

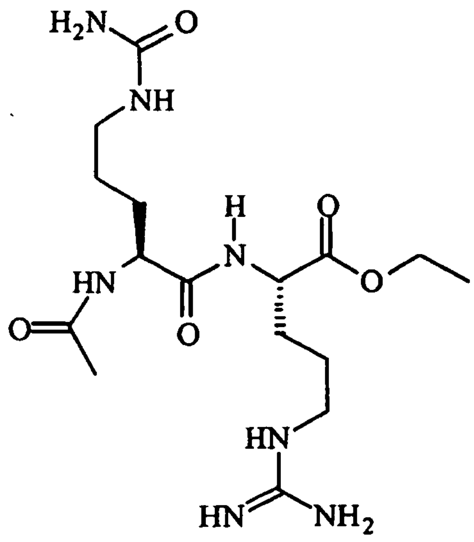

N-acetyl-L-citrullinyl-L-arginine (NAc-L-CIT-L-ARG)

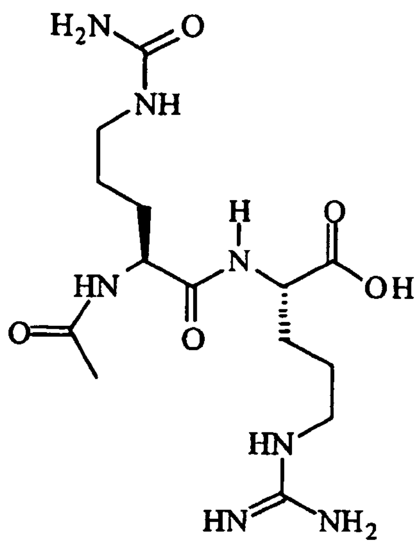

Comme il a été déjà mentionné, la molécule citrullinylarginine purifiée présente notamment une activité sur le métabolisme énergétique cellulaire, des propriétés cytostimulantes ainsi qu'une action potentialisatrice sur l'expression des protéines chaperonnes visant à améliorer le comportement d'une peau ou de phanères soumis au froid.

Plusieurs tests in vitro ont été réalisés afin de comparer les activités du dipeptide naturel purifié ci-dessus évoquées avec celles de l'adduit algal, objet de l'invention, et de certains analogues chimiques.

Les tests suivants illustrent ces données comparatives.

Test 1 : influence d'analogues du dipeptide naturel citrullinylarginine sur le métabolisme énergétique de fibroblastes en culture par un dosage de l'adénosine triphosphate (ATP). Comparaison avec le dipeptide naturel purifié.

Les cellules de fibroblastes sont cultivées et ensemencées au taux de $10^5$ cellules/ml en présence d'un milieu appauvri en facteur de croissance (2% de sérum de veau foetal).
Sur chaque suspension cellulaire, la quantité d'ATP est mesurée par photométrie, en déterminant la décroissance de l'absorbance à 340 nm, selon les équations suivantes : ATP + 3-phosphoglycérate ----> ADP + 1,3-disphosphoglycérate 1,3-diphosphoglycérate + NADH ----> glycéraldéhyde-3-P + NAD + P

Résultats :

| | quantité d'ATP (nM/mg protéines) |
|---|---|
| A (témoin sans actif) | 26.7 +/- 5.2 |
| A + Cit-Arg purifié 1% | 88.62 +/- 8 |
| A + adduit algal 1% | 96.2 +/- 6.2 |
| A + NAc-Cit-Arg 1% | 90.7 +/- 6.5 |

Test 2 : démonstration des propriétés cytostimulantes d'analogues du dipeptide naturel citrullinylarginine. Comparaison avec le dipeptide naturel purifié.

Le test a été réalisé sur une lignée cellulaire de fibroblastes humains maintenus dans un milieu de culture complété avec 2% de de sérum de veau foetal (témoin).
L'évaluation de la prolifération cellulaire est effectuée par un test colorimétrique au rouge neutre (Borenfreund E. et al., (1984), Toxicol. Lett., vol.24, pp.119). La variation de croissance cellulaire est alors obtenue en mesurant la densité optique à 540 nm, selon l'équation suivante :

$$\%\ \text{stimulation} = \frac{DO_{actif} - DO_{témoin}}{DO_{témoin}} * 100$$

Résultats :

| | | | | | |
|---|---|---|---|---|---|
| Cit-Arg purifié (%) | 0,016 | 0,031 | 0,063 | 0,125 | 0,5 |
| % stimulation | 2 | 25 | 30 | 32.5 | 36 |
| adduit algal (%) | 0,016 | 0,031 | 0,063 | 0,125 | 0,5 |
| % stimulation | 8 | 32 | 38 | 44 | 52 |
| NAc-Cit-Arg (%) | 0,016 | 0,031 | 0,063 | 0,125 | 0,5 |
| % stimulation | 5 | 26 | 34 | 40 | 45 |

Test 3 : action d'analogues du dipeptide naturel citrullinylarginine sur l'expression au froid des protéines chaperonnes (HSP 72). Comparaison avec le dipeptide naturel purifié.

Des épidermes reconstitués humains ont été soumis à une température de 4°C, en l'absence d'actif (témoin) puis en présence d'actif à tester. L'évaluation de l'expression des HSP 72 a été détectée semi-quantitativement par

immunofluorescence en utilisant un anticorps monoclonal spécifique de HSP 72. Elle repose sur une évaluation globale de la fluorescence observée. Plus la fluorescence est marquée, plus les protéines HSP 72 sont exprimées.

Résultats :

| | témoin | Cit-Arg purifié | adduit algal | NAc-Cit-Arg |
|---|---|---|---|---|
| HSP 72 | ++ | +++ | ++++ | +++ |
| ++ : quelques cellules sont fluorescentes<br>+++ : de nombreuses cellules sont fluorescentes<br>++++ : la totalité des couches cellulaires vivantes émet une fluorescence | | | | |

L'intérêt du dipeptide ou de ses analogues chimiques pour répondre au besoin d'une peau soumis à une luminosité réduite a été démontré par la réalisation d'un test in vivo sur un panel d'hommes et de femmes. Le test a été réalisé avec l'adduit algal présent dans la composition d'une crème détaillée ci-après, en combinaison avec des séances de luminothérapie.

Test 4 : action cutanée d'une crème formulée avec 7% d'extrait algal en combinaison avec des traitements de luminothérapie

La technique d'évaluation d'une telle action au niveau cutané a été celle de l'autoévaluation par échelle analogique.
Deux séries de séances de luminothérapie ont été ainsi menées :

- première série : dix personnes notent chacun des paramètres définis par la demanderesse, avant puis après un nombre précis de séances dans les conditions d'exposition suivantes :
  Eclairement = 2500 Lux
  Temps d'exposition = 20 minutes/jour
  Durée traitement = 2 fois/semaine pendant un mois
- deuxième série : les mêmes dix personnes notent les mêmes paramètres, après application topique d'une crème formulée avec 7% d'extrait algal, dans les mêmes conditions d'exposition.

Formulation de la composition contenant ledit extrait :

| | |
|---|---|
| glyceryl stéarate et steareth | 10 |
| cetearylethylhexanoate | 10 |
| macadamia ternilolia seed oil | 10 |
| glycerol | 3 |
| diméthicone | 0,3 |
| methylparaben sodé | 0,1 |
| propylparaben | 0,05 |
| imidazolidinylurée | 0,3 |
| polyacrylamide and C13-14 isoparaffin and laureth 7 | 1 |
| *Chondrus* extract (titré à 10% en citrullinylarginine) | 7 |
| eau purifiée qsp | 58,05 |

Résultats :

Il ressort, pour 80% des sujets, une amélioration significative de l'hydratation engendrant un toucher uniforme et agréable, pour 70% un retour à un éclat du teint, en particulier du visage, intégré avec les couches profondes dermiques

réduisant l'atonicité de la peau.

L'invention a également pour objet l'utilisation de l'adduit algal dans ou pour la préparation de compositions à usage dermatologique non-thérapeutique.

## Revendications

1. Adduit algal extrait de macroalgues rouges, **caractérisé en ce qu'**il est dépourvu de potentiel génotoxique et susceptible d'être obtenu par un procédé comprenant les étapes suivantes dans l'ordre suivant:

    - extraction desdites macroalgues rouges comprenant le dipeptide naturel citrullinylarginine au moyen d'un solvant ou d'un mélange de solvants acceptables en pharmacie;
    - enrichissement de la quantité en poids de polypeptides ou protéines intrinsèques par des protéines natives intrinsèques ou des protéines extrinsèques standards utilisables en pharmacie jusqu'à une teneur totale au moins égale à 20%;
    - éventuellement incorporation d'une teneur titrée du dipeptide citrullinylarginine obtenu par voie chimique;
    - traitement à chaud de l'extrait résultant.

2. Adduit selon la revendication 1, **caractérisé en ce que** ladite algue est une algue ayant accumulée de façon optimale 10% en poids sec de citrullinylarginine par rapport au poids total d'algue sèche.

3. Adduit selon la revendication 1 ou 2, **caractérisé en ce que** ladite extraction est réalisée au reflux dudit solvant ou mélange de solvants pendant une durée de 2 à 4 heures.

4. Adduit selon l'une quelconque des revendications 1 à 3, **caractérisé en ce que** ledit solvant ou mélange de solvants est un mélange d'eau et d'une solution éthanolique ammoniacale.

5. Adduit selon l'une quelconque des revendications 1 à 4, **caractérisé en ce que** ladite étape d'enrichissement résulte d'une concentration desdits polypeptides ou protéines intrinsèques.

6. Adduit selon l'une quelconque des revendications 1 à 4, **caractérisé en ce que** ladite étape d'enrichissement résulte de l'incorporation d'une quantité de protéines standards utilisables en pharmacie.

7. Adduit selon la revendication 6, **caractérisé en ce que** les protéines standards utilisables en pharmacie sont choisies parmi les protéines végétales, les protéines animales et les protéines algales, ou leurs hydrolysats respectifs, de préférence les protéines de blé ou de soja, le collagène, l'élastine, le collagène marin, la spiruline, les protéines microalgales

8. Adduit selon l'une quelconque des revendications 1 à 7, **caractérisé en ce que** l'étape de traitement est réalisée durant 3 heures à 40 DEG C.

9. Utilisation non-thérapeutic de l'adduit selon l'une quelconque des revendications 1 à 8 et 20 comme agent de soin et de traitement pour la peau et les phanères.

10. Utilisation de l'adduit selon la revendication 9 comme agents de soin et de traitement destinés à être une source potentielle d'azote.

11. Utilisation de l'adduit selon la revendication 9 comme agent de soin et de traitement permettant d'activer le métabolisme énergétique cellulaire.

12. Utilisation de l'adduit selon la revendication 9 comme agent de soin et de traitement présentant des propriétés cytostimulantes.

13. Utilisation de l'adduit selon la revendication 9 comme agent de soin et de traitement permettant d'améliorer le comportement d'une peau ou de phanères soumis au froid.

14. Procédé d'obtention d'un adduit algal de macroalgues rouges dépourvu de potentiel génotoxique **caractérisé en ce qu'**il comprend les étapes suivantes, dans l'ordre suivant :

a. extraction desdites macroalgues rouges comprenant le dipeptide naturel citrullinylarginine au moyen d'un solvant ou d'un mélange de solvants acceptables en pharmacie;
b. enrichissement de la quantité en poids de polypeptides ou protéines intrinsèques par des protéines natives intrinsèques ou des protéines extrinsèques standards utilisables en pharmacie jusqu'à une teneur totale au moins égale à 20%;
c. éventuellement incorporation d'une teneur titrée du dipeptide citrullinylarginine obtenu par voie chimique;
d. traitement à chaud de l'extrait résultant.

**15.** Procédé selon la revendication selon la revendication 14, **caractérisé en ce que** ladite extraction est réalisée au reflux dudit solvant ou mélange de solvants pendant une durée de 2 à 4 heures.

**16.** Procédé selon la revendication 14 ou 15, **caractérisé en ce que** ledit solvant ou mélange de solvants est un mélange d'eau et d'une solution éthanolique ammoniacale.

**17.** Procédé selon l'une quelconque des revendications 14 à 16, **caractérisé en ce que** ladite étape d'enrichissement résulte d'une concentration desdits polypeptides ou protéines intrinsèques.

**18.** Procédé selon l'une quelconque des revendications 14 à 17, **caractérisé en ce que** ladite étape d'enrichissement résulte de l'incorporation d'une quantité de protéines standards utilisables en pharmacie.

**19.** Procédé selon l'une quelconque des revendications 14 à 18, **caractérisé en ce que** l'étape de traitement est réalisée durant 3 heures à 40 DEG C.

**20.** Adduit selon l'une quelconque des revendications 1 à 8, **caractérisé en ce que** la macroalgue rouge est Chondrus crispus.

**Claims**

**1.** Algal adduct extracted from red macroalgae, **characterized in that** it is devoid of genotoxic potential and likely to be obtained through a process including the following steps, in the following order :

- extraction of said macroalgae containing the citrullinylarginine natural dipeptide with a solvent or a mixture of solvents usable in pharmacy ;
- enrichment of the weight amount of intrinsic polypeptides or proteins by intrinsic native proteins or extrinsic standard proteins usable in pharmacy up to a total content equal to at least 20% ;
- possibly incorporation of a titrated content in citrullinylarginine dipeptide obtained by chemical way,
- heat treatment of the resulting extract.

**2.** Adduct according to claim 1, **characterized in that** the said alga is an alga having stored optimally 10% in dry weight of citrullinylarginine in relation to the total weight of dried alga.

**3.** Adduct according to claims 1 or 2, **characterized in that** the said extraction is performed under reflux of said solvent or mixture of solvents during 2 to 4 hours.

**4.** Adduct according to one of claims 1 to 3, **characterized in that** the said solvent or mixture of solvents is a mixture of water and an ethanol solution of ammonia.

**5.** Adduct according to one of claims 1 to 4, **characterized in that** the said enrichment step results from a concentration of said intrinsic polypeptides or proteins.

**6.** Adduct according to one of claims 1 to 4, **characterized in that** the said enrichment step results from the incorporation of an amount of standard proteins usable in pharmacy.

**7.** Adduct according to claim 6, **characterized in that** the standard proteins usable in pharmacy are chosen among plant proteins, animal proteins and algal proteins, or their respective hydrolysates, preferably wheat or soya proteins, collagen, elastin, marine collagen, spiruline, microalgal proteins.

**8.** Adduct according to one of claims 1 to 7, **characterized in that** the treatment step is performed during 3 hours at 40°C.

**9.** Non-therapeutic use of the adduct according to one of claims 1 to 8 and 20 as a skin and skin appendages care and treatment agent.

**10.** Use of the adduct according to claim 9 as a care and treatment agent intented to be a potential source of nitrogen.

**11.** Use of the adduct according to claim 9 as a care and treatment agent able to activate the cellular energy metabolism.

**12.** Use of the adduct according to claim 9 as a care and treatment agent displaying cytostimulating properties.

**13.** Use of the adduct according to claim 9 as a care and treatment agent able to improve the behaviour of skin or skin appendages exposed to cold.

**14.** Process of obtaining of an algal adduct from red macroalgae devoid of genotoxic potential **characterized in that** it comprises the following steps, in the following order :

a. extraction of said red macroalgae containing the citrullinylarginine natural dipeptide with a solvent or a mixture of solvents usable in pharmacy ;
b. enrichment of the weight amount of intrinsic polypeptides or proteins by intrinsic native proteins or extrinsic standard proteins usable in pharmacy up to a total content equal to at least 20% ;
c. possibly incorporation of a titrated content in citrullinylarginine dipeptide obtained by chemical way ;
d. heat treatment of the resulting extract.

**15.** Process according to claim 14, **characterized in that** the said extraction is performed under reflux of said solvent or mixture of solvents during 2 to 4 hours.

**16.** Process according to claims 14 or 15, **characterized in that** the said solvent or mixture of solvents is a mixture of water and an ethanol solution of ammonia.

**17.** Process according to one of claims 14 to 16, **characterized in that** the said enrichment step results from a concentration of said intrinsic polypeptides or proteins.

**18.** Process according to one of claims 14 to 17, **characterized in that** the said enrichment step results from the incorporation of an amount of standard proteins usable in pharmacy.

**19.** Process according to one of claims 14 to 18, **characterized in that** the treatment step is performed during 3 hours at 40°C.

**20.** Adduct according to one of claims 1 to 8, **characterized in that** the red macroalga is Chondrus crispus.

## Patentansprüche

**1.** Ein Algenaddukt, extrahiert aus roten Makroalgen,
**dadurch gekennzeichnet, dass** es kein genotoxisches Potenzial besitzt und mittels eines Verfahrens erhalten werden kann, das die folgenden Schritte in der folgenden Reihenfolge umfasst:

- Extrahieren der roten Makroalgen, umfassend das natürliche Citrullinylarginin-Dipeptid, mittels eines Lösungsmittels oder einer Mischung aus pharmazeutisch annehmbaren Lösungsmitteln;
- Anreichern der Gewichtsmenge von Polypeptiden oder intrinsischen Proteinen mit intrinsischen nativen Proteinen oder extrinsischen Standardproteinen, die pharmazeutisch verwendbar sind, bis zu einem Gesamtgehalt von mindestens 20 %;
- gegebenenfalls Beimischen eines Gehalts, der mit dem Citrullinylarginin-Dipeptid, das chemisch erhalten wurde, titriert wurde;
- Wärmebehandeln des resultierenden Extrakts.

**2.** Addukt gemäß Anspruch 1, **dadurch gekennzeichnet, dass** die Alge eine Alge ist, die gegenüber dem Gesamtgewicht an Trockenalge optimalerweise 10 Trockengew.-% Citrullinylarginin akkumuliert hat.

**3.** Addukt gemäß Anspruch 1 oder 2, **dadurch gekennzeichnet, dass** das Extrahieren während des Rückflusses des Lösungsmittels oder der Mischung aus Lösungsmitteln über einen Zeitraum von 2 bis 4 Stunden erfolgt.

**4.** Addukt gemäß einem der Ansprüche 1 bis 3, **dadurch gekennzeichnet, dass** das Lösungsmittel oder die Mischung aus Lösungsmitteln eine Mischung aus Wasser und einer ammoniakartigen Ethanollösung ist.

**5.** Addukt gemäß einem der Ansprüche 1 bis 4, **dadurch gekennzeichnet, dass** der Anreicherungsschritt aus einer Konzentration der Polypeptide oder intrinsischen Proteine resultiert.

**6.** Addukt gemäß einem der Ansprüche 1 bis 4, **dadurch gekennzeichnet, dass** der Anreicherungsschritt aus der Beimischung einer Menge an pharmazeutisch verwendbaren Standardproteinen resultiert.

**7.** Addukt gemäß Anspruch 6, **dadurch gekennzeichnet, dass** die pharmazeutisch verwendbaren Standardproteine aus Pflanzenproteinen, Tierproteinen und Algenproteinen oder deren jeweiligen Hydrolysaten, vorzugsweise Weizen- oder Sojaproteinen, Kollagen, Elastin, Meereskollagen, Spirulina, Mikroalgenproteinen ausgewählt sind.

**8.** Addukt gemäß einem der Ansprüche 1 bis 7, **dadurch gekennzeichnet, dass** der Behandlungsschritt über einen Zeitraum von 3 Stunden bei 40 °C erfolgt.

**9.** Eine nichttherapeutische Verwendung des Addukts gemäß einem der Ansprüche 1 bis 8 und 20 als Pflege- und Behandlungsmittel für die Haut und Hautanhangsgebilde.

**10.** Verwendung des Addukts gemäß Anspruch 9 als Pflege- und Behandlungsmittel, das als eine potenzielle Stickstoffquelle bestimmt ist.

**11.** Verwendung des Addukts gemäß Anspruch 9 als Pflege- und Behandlungsmittel, das die Aktivierung des energetischen Zellstoffwechsels ermöglicht.

**12.** Verwendung des Addukts gemäß Anspruch 9 als Pflege- und Behandlungsmittel, das zytostimulierende Eigenschaften aufweist.

**13.** Verwendung des Addukts gemäß Anspruch 9 als Pflege- und Behandlungsmittel, das das Verhalten einer Haut oder von Hautanhangsgebilden gegenüber Kälte verbessern kann.

**14.** Ein Verfahren zum Erhalten eines Algenaddukts aus roten Makroalgen, das kein genotoxisches Potenzial aufweist, **dadurch gekennzeichnet, dass** es die folgenden Schritte in der folgenden Reihenfolge umfasst:

a. Extrahieren der roten Makroalgen, umfassend das natürliche Citrullinylarginin-Dipeptid, mittels eines Lösungsmittels oder einer Mischung aus pharmazeutisch annehmbaren Lösungsmitteln;
b. Anreichern der Gewichtsmenge von Polypeptiden oder intrinsischen Proteinen mit intrinsischen nativen Proteinen oder extrinsischen Standardproteinen, die pharmazeutisch verwendbar sind, bis zu einem Gesamtgehalt von mindestens 20 %;
c. gegebenenfalls Beimischen eines Gehalts, der mit dem Citrullinylarginin-Dipeptid, das chemisch erhalten wurde, titriert wurde;
d. Wärmebehandeln des resultierenden Extrakts.

**15.** Verfahren gemäß Anspruch 14, **dadurch gekennzeichnet, dass** das Extrahieren während des Rückflusses des Lösungsmittels oder der Mischung aus Lösungsmitteln über einen Zeitraum von 2 bis 4 Stunden erfolgt.

**16.** Verfahren gemäß einem der Ansprüche 14 oder 15, **dadurch gekennzeichnet, dass** das Lösungsmittel oder die Mischung aus Lösungsmitteln eine Mischung aus Wasser und einer ammoniakartigen Ethanollösung ist.

**17.** Verfahren gemäß einem der Ansprüche 14 bis 16, **dadurch gekennzeichnet, dass** der Anreicherungsschritt aus einer Konzentration der Polypeptide oder intrinsischen Proteine resultiert.

**18.** Verfahren gemäß einem der Ansprüche 14 bis 17, **dadurch gekennzeichnet, dass** der Anreicherungsschritt aus

der Beimischung einer Menge an pharmazeutisch verwendbaren Standardproteinen resultiert.

**19.** Verfahren gemäß einem der Ansprüche 14 bis 18, **dadurch gekennzeichnet, dass** der Behandlungsschritt über einen Zeitraum von 3 Stunden bei 40 °C erfolgt.

**20.** Addukt gemäß einem der Ansprüche 1 bis 8, **dadurch gekennzeichnet, dass** die rote Makroalge Chondrus crispus ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2655268
- WO 9409750 A
- EP 1060739 A1


### Littérature non-brevet citée dans la description


- Nitrogen in the marine environment. Academic press
- **Miyazawa K. et al.** *Bulletin of the Japonese Society of Scientific Fisheries,* 1974, vol. 40, 815-818
- **Laycock M.V. et al.** *Can J. Biochem.,* 1977, vol. 55, 27-30
- **Laycock M.V. et al.** *Can J. Biochem.,* 1981, vol. 59, 522-527
- **Chyun J.H. et al.** *J. Nutr.,* 1984, vol. 114, 1697-1704
- **Holland D.B. et al.** *J. Invest. Dermatology,* 1993, vol. 101, 196-199
- **Chithra P. et al.** *J. Clin. Biochem. Nutr.,* 1995, vol. 18, 111-117
- **Kubilus J. et al.** *Biochim. Biophys. Acta,* 1979, vol. 581, 114-121
- **Rogers G.E. et al.** *Biochim. Biophys. Acta,* 1977, vol. 495, 159-175
- **Wohlrab J.** *Skin Pharmacol. Appl. Skin Physiol.,* 2002, vol. 15, 44-54
- **Kirkland D.J.** *Mutation Research,* 1994, vol. 312, 195-199
- **Borenfreund E. et al.** *Toxicol. Lett.,* 1984, vol. 24, 119